# EUROPEAN PATENT APPLICATION

(11) **EP 4 257 685 A1**
(43) Date of publication of application: **11.10.2023**
(21) Application number: 22167222.3
(22) Date of filing: 07.04.2022
(51) Int. Cl.: C12N 15/10

(54) **METHODS FOR THE REMOVAL OF DOUBLE- AND/OR MULTI-STRANDED NUCLEIC ACID IMPURITIES FROM RNA PREPARATIONS BY LOW PH TREATMENT**

(71) Applicant: Sartorius BIA Separations d.o.o., 5270 Ajdovscina (SI)
(72) Inventor: Puc, Jasmina, 5000 Nova Gorica (SI); Megusar, Polona, 4228 Zelezniki (SI); Sekirnik, Rok, 1370 Logatec (SI)
(74) Representative: Müller-Boré & Partner Patentanwälte PartG mbB

(57) **Abstract**

The present invention relates to methods for the removal of double- and/or multi-stranded nucleic acid impurities from an RNA preparation, comprising the steps of incubating the RNA preparation at a pH in the range of pH 1 to pH 5, and subjecting the RNA preparation to purification to remove fragments produced by the dissociation of the double- and/or multi-stranded nucleic acid impurities.

## Description

The present invention relates to methods for the removal of double- and/or multi-stranded nucleic acid impurities from an RNA preparation, comprising the steps of incubating the RNA preparation at a pH in the range of pH 1 to pH 5, and subjecting the RNA preparation to purification to remove fragments produced by the dissociation of the double- and/or multi-stranded nucleic acid impurities.

Recent clinical success of mRNA-based COVID-19 vaccines triggered an unprecedented investment into development of RNA-based therapeutics as vaccines in multiple therapeutic areas.

RNA is produced by enzymatic conversion of DNA template to RNA in *in vitro* transcription (IVT) reaction, using RNA polymerase enzyme. RNAs used for therapeutic application include messenger RNA (mRNA), self-amplifying RNA (saRNA) and circular RNA. Impurities produced by IVT reaction include a range of double- and multi-stranded nucleic acid species, a major impurity being double-stranded RNA (dsRNA). dsRNA is a critical impurity in RNA therapeutics due to possible immunogenic responses, wherein the host immune system recognizes dsRNA as invading virus. Cellular innate immune response to dsRNA by-products can lead to undesirable consequences, including suppression of protein synthesis and cell death, which in turn can detrimentally impact the efficacy of RNA therapy.

Two main types of dsRNA by-products have been identified: one is formed by 3'-extension of the run-off products annealing to complementary sequences in the body of the run-off transcript either in *cis* (by folding back on the same RNA molecule) or *trans* (annealing to a second RNA molecule) to form extended duplexes. The second type of dsRNA is formed by hybridization of antisense RNA molecule to the run-off transcript. Antisense RNA molecules have been reported to be formed in a promoter- and run-off transcript-independent manner. RNA polymerase might switch to the non-template strand, resulting in an RNA molecule that is complementary to the runoff product.

dsRNA thus forms a heterogeneous population of RNA sequences of different lengths, rather than a well-defined impurity.

dsRNA removal is typically performed by one of two chromatographic approaches, both utilizing differences in relative hydrophobicity of single stranded (ssRNA) versus dsRNA. The first of these approaches is reverse phase (RP) HPLC, and the second is chromatography using chromatographic cellulose media. However, these current approaches are non-specific and poorly scalable. In particular, RP-chromatography utilizes high temperatures and organic solvents, both of which are undesirable in the manufacturing of biological therapeutics. Further, they remove 3'-extended products, as those are longer and consequently more hydrophobic than ssRNA, but not antisense transcripts produced as run-off transcripts. Moreover, cellulose as a chromatographic medium is poorly defined and not easily scalable. Further, the forces driving the separation are not understood and therefore not well controlled.

A third approach for removal of dsRNA is enzymatic degradation using RNAse III. However, RNAse III is non-specific, *i.e.,* it digests dsRNA as well as ssRNA. Further, the use of enzymes in the production process is undesirable, so this approach is not feasible for commercial-scale manufacturing of RNA.

A second important class of impurities related to RNA preparations is residual DNA template used for *in vitro* transcription to RNA. DNA template is typically a linearized form of DNA encoding the gene of interest, 5' and 3' UTR regions, polymerase binding sites, and selection markers. The presence of residual plasmid template in RNA preparations could lead to immunogenic responses, so that residual plasmid must be removed in from the final RNA preparation. Typical purification approaches include DNAse treatment post-IVT, coupled with a purification step (precipitation, tangential flow filtration (TFF) or chromatography). Although highly selective and efficient, this approach introduces additional contaminants (e.g. enzymes), which in turn need to be removed in the purification process. In absence of DNAse treatment, affinity purification of RNA (e.g. Oligo dT-purification of polyadenylated mRNA) achieves a high, but not complete, level of DNA removal, where residual plasmid after affinity purification is presumed to be interacting with RNA molecules via specific or non-specific base-pair interactions (forming what is defined herein as double- and/or multi-stranded nucleic acid impurities).

Accordingly, the technical problem underlying the present invention is the provision of improved means for the removal of double- and/or multi-stranded nucleic acid impurities from RNA preparations, which should exhibit a high specificity, good scalability, and, therefore, be suitable for the manufacturing of RNA-based therapeutics at commercially feasible scales.

The solution to the above technical problem is achieved by the embodiments characterized in the claims.

In particular, the present invention relates to a method for the removal of double- and/or multi-stranded nucleic acid impurities from an RNA preparation, comprising the steps of:
(a) incubating the RNA preparation at a pH in the range of pH 1 to pH 5, and
(b) subjecting the RNA preparation to purification to remove fragments produced by the dissociation of the double- and/or multi-stranded nucleic acid impurities.

Double- and/or multi-stranded nucleic acid impurities that can be relevant in the preparation of RNA and that can be removed according to the present invention include any non-single-stranded nucleic acid structures, in particular dsRNA, dsDNA (e.g. template DNA from IVT reaction), RNA-DNA heteroduplex structures, and multi-stranded homo- or heteromeric nucleic acid structures, e.g. formed by the unspecific (i.e., non-Watson-Crick based) association of RNA strands and/or DNA strands (e.g. RNA/DNA triplexes). In this context, the term "removal of double- and/or multi-stranded nucleic acid impurities" expressly includes the situation wherein a contaminating RNA or DNA single strand, or DNA double strand, has annealed to a target RNA (i.e., an RNA that is to be prepared in the RNA preparation), and the resulting dsRNA structure or RNA-DNA heteroduplex or RNA-DNA triplex or other multiplex structures are subsequently removed by causing the dissociation of the contaminating RNA or DNA strand(s) from the target RNA.

According to the present invention, an RNA preparation containing double- and/or multi-stranded nucleic acid impurities is subjected to low pH conditions, in the range of pH 1 to pH 5, which causes the dissociation of the respective nucleic acid strands from each other.

The term "RNA preparation" as used herein relates to any physical solution containing a target RNA in a suitable buffer. Incubation of the RNA preparation at a pH in the range of pH 1 to pH 5 includes any means of providing a respective pH environment to the target RNA, e.g., by way of acidifying the buffer in which the target RNA is present (e.g., by way of combining the buffer with an acidic buffer), or by way of transferring the target RNA into a new (acidic) buffer.

According to the present invention, the above RNA preparation is incubated at a pH in the range of pH 1 to pH 5, which range expressly includes the boundary values pH 1 and pH 5. In preferred embodiments, the RNA preparation is incubated at a pH in a range, the lower boundary of which is selected from the group consisting of pH 1.0, pH 1.1, pH 1.2, pH 1.3, pH 1.4, pH 1.5, pH 1.6, pH 1.7, pH 1.8, pH 1.9, and pH 2.0, and the upper boundary of which is selected from the group consisting of pH 5.0, pH 4.9, pH 4.8, pH 4.7, pH 4.6, pH 4.5, pH 4.4, pH 4.3, pH 4.2, pH 4.1, pH 4.0, pH 3.9, pH 3.8, pH 3.7, pH 3.6, pH 3.5, pH 3.4, pH 3.3, pH 3.2, pH 3.1, and pH 3.0. In specific embodiments, the RNA preparation is incubated at a pH in the range of pH 2 to pH 4, in the range of pH 2 to pH 3.5, or in the range of pH 2 to pH 3. For example, the RNA preparation can be incubated at about pH 3.

Incubation in step (a) of the method of the present invention can be done for an amount of time that is sufficient for allowing dissociation of a majority of nucleic acid strands from each other. Respective durations depend on a variety of factors, including temperature, pH, and the nature of the impurities (e.g. with respect to the length of the double- and/or multi-stranded region), and can be suitably chosen by the person skilled in the art. Respective durations range from a few seconds (including e.g. one second) to several hours, where exemplary duration ranges include incubation for 25 seconds to 1 hour, 1 minute to 1 hour, 10 to 40 minutes, 20 to 40 minutes, 25 to 35 minutes, e.g., about 30 minutes.

In specific embodiments, step (a) of the method of the present invention is performed in an acidic buffer. Suitable buffers for any chosen pH or pH range are known in the art and can be chosen or designed by the person skilled in the art. Exemplary buffers in this respect include buffers having a pKₐ in the range of 2 to 5. Specific exemplary buffers include citrate buffers, acetate buffers, glycine buffers, glycylglycine buffers, malate buffers, maleate buffers, phosphate buffers, succinate buffers, formate buffers, and combinations thereof. Respective buffers can contain a denaturing agent and/or a chelating agent, wherein the denaturing agent is preferably a chaotropic agent, an organic solvent, and combinations thereof. Respective chaotropic agents, organic solvents, and chelating agents are not particularly limited and are known in the art. Such chaotropic agents include e.g. urea, guanidine, and combinations thereof. Further, organic solvents include e.g. acetonitrile. Furthermore, chelating agents include e.g., EDTA (ethylenediaminetetraacetic acid).

Step (a) of the method of the present invention is preferably performed at a temperature of 4°C to 50°C, e.g., at 8°C to 40°C, 15°C to 30°C, or 20°C to 25°C, e.g., at about 20°C or at about 25°C. In particular, step (a) can be performed at ambient temperature. Alternatively, step (a) can be performed at elevated temperatures with respect to ambient temperature, e.g., at a temperature of 25°C to 50°C, 25°C to 40°C, or 30°C to 40°C. In any case, step (a) of the method of the present invention is not performed at temperatures that by itself would cause the dissociation of double- and/or multi-stranded nucleic acid impurities, e.g., the method is not performed at temperatures higher than 50°C.

In specific embodiments, step (a) of the method of the present invention is performed as part of an affinity chromatography step, in which the RNA preparation is loaded onto the affinity chromatography medium at a pH in the range of pH 6 to pH 8, e.g., at a pH of about 7, and subsequently the pH is lowered to a pH in the range of pH 1 to pH 5, thus fulfilling step (a) of the method of the present invention. Suitable affinity ligands in this respect are not particularly limited and are known in the art. They include nucleic acids that are complementary to the target RNA, e.g., oligo-dT.

In step (b) of the method of the present invention, the RNA preparation is subjected to purification to remove fragments produced by the dissociation of the double- and/or multi-stranded nucleic acid impurities.

The fragments produced by the dissociation of the double- and/or multi-stranded nucleic acid impurities, *i.e.,* produced by the dissociation of the strands of the double- and/or multi-stranded nucleic acid impurities from each other, that are to be removed in this step (b) are contaminating single strand RNAs that are different from the target RNA, or contaminating single strand DNAs. Means for effecting a respective purification of the RNA preparation are not particularly limited and are known in the art. However, it is preferred that such means are as defined below.

In specific embodiments, the RNA preparation is subjected to the purification at the pH used in step (a), *i.e.,* the pH is not adjusted prior to the purification. In other specific embodiments, the pH of the RNA preparation is increased to a pH higher than the pH used in step (a), wherein the pH is, however, not higher than pH 5, including pH 5, prior to the purification. These embodiments for obvious reasons do not apply in case step (a) is already performed at pH 5. In yet other specific embodiments, the pH of the RNA preparation is increased to a pH in the range of more than pH 5 to pH 7, including pH 7, prior to the purification.

In specific embodiments, step (a) of the methods of the present invention is performed at a pH in the range of pH 2 to pH 4, e.g., at about pH 3, and subsequently, prior to purification in step (b), the pH is increased to a pH in the range of pH 4.5 to pH 5, e.g., to pH 5.

As described so far herein, steps (a) and (b) are separate steps that are performed subsequently in the order of first performing step (a), and then performing step (b). Thus, in specific embodiments, steps (a) and (b) are separate steps that are performed subsequently in this indicated order. As indicated above, in such embodiments, step (b) can be performed at a pH that is the same as the pH used in step (a), or the pH can be increased prior to step (b).

However, in other specific embodiments, steps (a) and (b) can be performed concurrently, *i*.*e*., the incubation of the RNA preparation at a pH in the range of pH 1 to pH 5 according to the step (a) can be a part of the purification process to remove fragments produced by the dissociation of the double- and/or multi-stranded nucleic acid impurities in step (b). By way of example, in the case of purification by chromatography-based techniques as described herein, step (a) of the methods of the present invention can be performed while the RNA preparation is bound to a chromatographic medium, and step (b) of the methods of the present invention is effected using the chromatographic medium. In such embodiments, pH denaturation according to the present invention is effected by binding the RNA preparation to a chromatographic medium at conditions that favour respective binding (e.g., at a pH in the range of pH 1 to pH 8, or at a pH in the range of pH 6 to pH 8, e.g. at a pH of about 7), the pH is then decreased to a pH range of pH 1 to pH 5 according to step (a) of the methods of the present invention to effect nucleic acid strand dissociation on the chromatographic medium, and subsequent or concurrent elution of dissociated ssRNA and nucleic acid impurities is performed e.g. by pH gradient, salt gradient or a combination thereof.

As indicated above, it is preferred that means for effecting a respective purification of the RNA preparation are as defined herein. Specifically, the purification can be effected by a technique, selected from the group consisting of cation exchange chromatography, anion exchange chromatography, size exclusion chromatography, reversed phase chromatography, hydrophobic interaction chromatography, multi-modal chromatography, affinity chromatography, IMAC (immobilized metal affinity chromatography), molecular weight cut-off filtration, and precipitation/extraction techniques. Means for performing such techniques are not particularly limited and are known in the art.

In the case of chromatographic techniques as indicated above, the chromatographic material can be in any suitable form, e.g., in the form of porous particles, membranes, nanofibers, filters, and monoliths as known in the art.

In a specific embodiment, purification is effected by anion exchange chromatography that is performed at a pH in the range of pH 1 to pH 5 using an anion exchange ligand. Respective anion exchange ligands are not particularly limited and are known in the art. Exemplary anion exchange ligands that can be used in this respect include quaternary amines (QA), as well as tertiary, secondary, and primary amines, wherein diethylaminoethyl (DEAE) and dimethylaminoethyl (DMAE) are particularly suitable. As indicated above, anion exchange chromatography is performed at a pH in the range of pH 1 to pH 5, which range expressly includes the boundary values pH 1 and pH 5, or at a pH in a preferred range as defined above for step (a) of the method of the present invention. In a related preferred embodiment, anion exchange chromatography is performed at the same pH as step (a) of the method of the present invention.

In another specific embodiment, purification is effected by cation exchange chromatography that is performed at a pH in the range of pH 1 to pH 5 using a cation exchange ligand. Respective cation exchange ligands are not particularly limited and are known in the art. Exemplary cation exchange ligands that can be used in this respect include cation exchange ligands containing sulfonate groups, cation exchange ligands containing sulfate groups, and cation exchange ligands containing carboxyl groups. As indicated above, cation exchange chromatography is performed at a pH in the range of pH 1 to pH 5, which range expressly includes the boundary values pH 1 and pH 5, or at a pH in a preferred range as defined above for step (a) of the method of the present invention. In a related preferred embodiment, cation exchange chromatography is performed at the same pH as step (a) of the method of the present invention.

In a further specific embodiment, purification is effected by multi-modal chromatography that is performed at a pH in the range of pH 1 to pH 5 using a multi-modal ligand, e.g., a ligand combining ion exchange/hydrogen bonding properties or a ligand combining ion exchange/aromatic moieties, or a combination thereof. Respective multi-modal ligands are not particularly limited and are known in the art. Exemplary multi-modal ligands that can be used in this respect include CIM PrimaS, CIM PrimaH, CIM H-bond, Capto MMC, Sartobind STIC, and Toyopearl NH2. In general, any kind of multi-modal ligand comprising multiple types of chemical residues selected from the group consisting of affinity residues, hydrophobic interaction residues, ion exchange residues, hydrogen bonding residues, metal chelating residues and aromatic residues and any combination thereof can be suitable for the methods of the present invention. As indicated above, multi-modal chromatography is performed at a pH in the range of pH 1 to pH 5, which range expressly includes the boundary values pH 1 and pH 5, or at a pH in a preferred range as defined above for step (a) of the method of the present invention. In a related preferred embodiment, multi-modal chromatography is performed at the same pH as step (a) of the method of the present invention.

In yet a further specific embodiment, purification is effected by affinity chromatography that is performed at a pH in the range of pH 1 to pH 5 using an affinity ligand, such as oligo dT or other sequence-specific nucleic acid ligands that are complementary to the target RNA. As indicated above, affinity chromatography is performed at a pH in the range of pH 1 to pH 5, which range expressly includes the boundary values pH 1 and pH 5, or at a pH in a preferred range as defined above for step (a) of the method of the present invention. In a related preferred embodiment, affinity chromatography is performed at the same pH as step (a) of the method of the present invention.

In alternative specific embodiments, purification is effected by
(i) size exclusion chromatography,
(ii) reversed phase chromatography,
(iii) hydrophobic interaction chromatography,
(iv) IMAC (immobilized metal affinity chromatography),
(iv) molecular weight cut-off filtration, or
(v) precipitation/extraction techniques,
that is/are performed at a pH in the range of pH 1 to pH 5, or at a pH in the range of more than pH 5 to pH 7. As indicated above, these purification techniques can be performed at a pH in the range of pH 1 to pH 5, which range expressly includes the boundary values pH 1 and pH 5, or at a pH in a preferred range as defined above for step (a) of the method of the present invention. In a related preferred embodiment, these purification techniques are performed at the same pH as step (a) of the method of the present invention. Alternatively, the above purification techniques can be performed at a pH in the range of more than pH 5 to pH 7, which range expressly includes the boundary value pH 7, *i*.*e*., the techniques can be performed after a step of adjusting the pH of the RNA preparation to a respective pH after step (a) and prior to step (b) of the method of the present invention.

The target RNA, *i.e.,* the RNA to be prepared in the RNA preparation used in the method of the present invention, can be selected from the group consisting of messenger RNA (mRNA), self-amplifying RNA (saRNA) and circular RNA (circRNA), wherein mRNA is particularly preferred.

As used herein, the term "comprising"/"comprises" expressly includes the terms "consisting essentially of"/"consists essentially of" and "consisting of"/"consists of", *i.e.,* all of the terms are interchangeable with each other herein.

Further, as used herein, the term "about" preferably represents a modifier of the specified value of ± 10%, more preferably ± 8%, ± 6%, ± 5%, ± 4%, ± 3%, ± 2%, ± 1%, or ± 0.5%. Thus, by way of example, the term "about 100" can include the ranges of 90 to 110, 92 to 108, 94 to 106, 95 to 105, 96 to 104, 97 to 103, 98 to 102, 99 to 101, or 99.5 to 100.5.

The present invention describes a method for the removal of double- and/or multi-stranded nucleic acid impurities from RNA preparations. The method applies the use of pH modulation to remove the impurities from RNA-based therapeutics, e.g., mRNA, saRNA, or circRNA. pH modulation is coupled with a purification step to remove single-stranded fragments produced upon denaturation of double- and/or multi-stranded starting material.

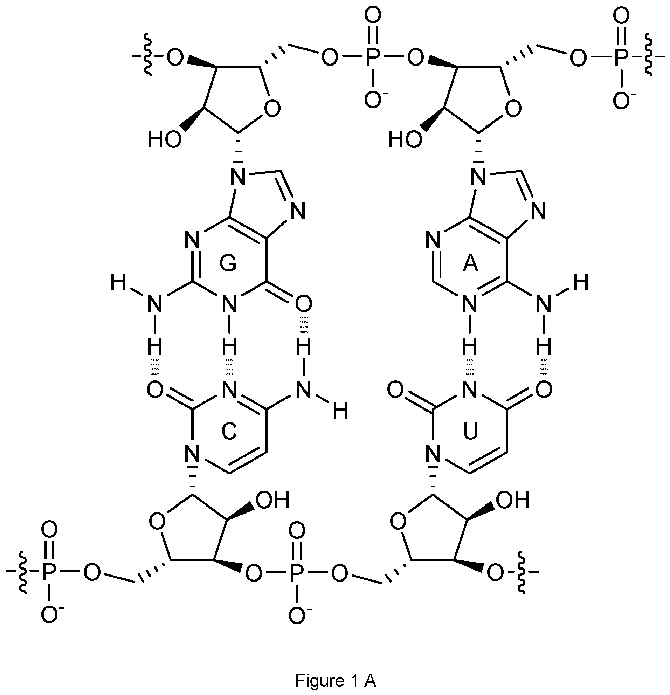

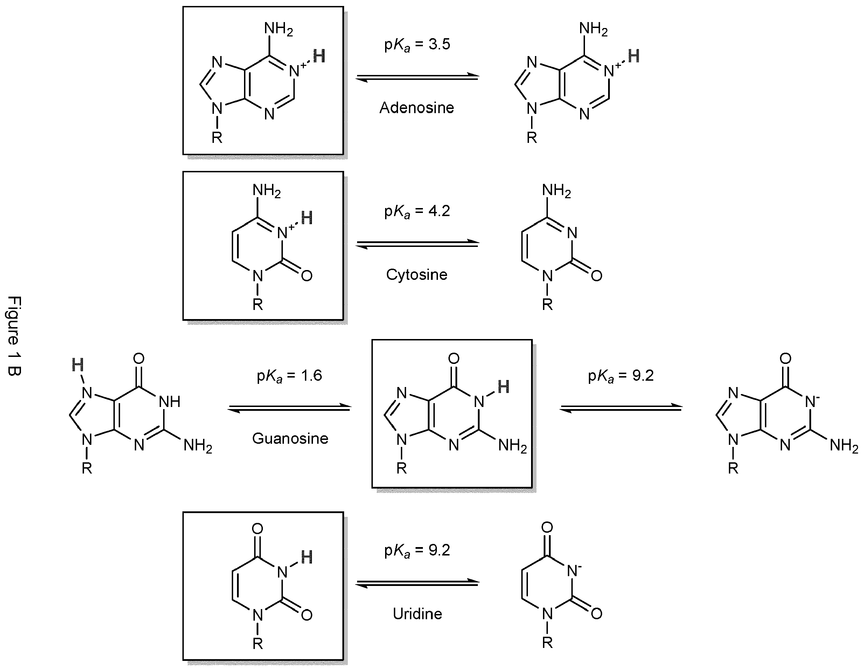

The formation of double- and/or multi-stranded nucleic acid structures, as well as the stability of the structures, depends on the fundamental principle of nucleic acid base-pairing. Formation is driven by canonical hydrogen bonding interactions between C-G and A-U base-pairs (Fig. 1). Correct formation of base pairs, and thus formation of dsRNA, is driven by formation of canonical bonding interactions, *i*.*e*., C-G and A-U. To form canonical interactions, guanosine requires N1 protonation (pH > 1.6), adenosine requires unprotonated N1 (pH > 3.5), cytosine requires unprotonated N3 (pH > 4.2), and uridine requires protonated N3 (pH < 9.2).

At pH ranges standardly used for RNA production, purification, and storage (*i.e*., pH 5 to 9), all four nucleosides are thus suitably protonated for base-pairing and consequently double strand formation. The phosphate backbone is deprotonated at pH > 1; therefore, RNA contains a molecular surface (phosphate backbone) that is negatively charged at working pH, a feature that is used in many purification approaches.

At pH ranges standardly used for RNA production, purification, and storage (i.e., pH 5 to 9), all four nucleosides are thus also suitably protonated for base-pairing with affinity ligands immobilized to chromatographic supports, e.g., poly-deoxythymidinic acid (Oligo dT). A decrease in pH in accordance with the present invention disrupts the base-pairing with affinity ligand, thus releasing the target RNA molecule, while simultaneously denaturing multi- and/or double-stranded interactions (RNA-RNA or RNA-DNA).

It has been demonstrated on short RNA duplexes (< 20 mers) that protonation of hydrogen bonding acceptors disrupts base-pairing and leads to denaturation of RNA duplexes, measured as a decrease in the melting temperature of RNA duplexes with decreasing pH. As a more general point, it was previously observed that low pH leads to denaturation (or at least destabilization) of nucleic acids due to protonation of G-C base pairs and resultant base pair formation. Furthermore, it was demonstrated that protonation stabilizes non-canonical interactions, e.g. A-C, and C-C in DNA duplexes. Denaturation of double-stranded DNA was shown by incubation in acidic conditions. However, this concept has never been demonstrated in the context of purification of biologics, where short RNA duplexes are not representative of therapeutically relevant RNA such as mRNA, and dsDNA is not representative of ssRNA contaminated with dsRNA, ssRNA, ssDNA or other double- and/or multi-stranded nucleic acid impurities.

Accordingly, the present invention concerns the denaturation of double- and/or multi-stranded nucleic acid impurities present in RNA preparations to single-stranded form through low pH-driven strand separation, leading to disruption of canonical base-pairing. Double- and/or multi-stranded nucleic acid impurities are disassociated from the RNA by incubation of the RNA preparation at ambient to mildly elevated temperatures at a pH range of pH 1 to pH 5, preferably pH 2 to pH 4. This pH range is sufficient for protonation of bases involved in base-pairing, rendering them unable to support canonical base-pairing. The phosphate backbone remains unprotonated, and RNA thus retains an overall negative charge. Citrates, acetates, glycine, or other acidic buffers may be used. Additives that facilitate denaturation, e.g. chaotropes, e.g. urea or guanidine, may be used to promote double and/or multi strand denaturation under acidic conditions. Other denaturants known in the art, e.g. acetonitrile may be used at low pH to aid denaturation. Chelators that facilitate removal of structural divalent cations, e.g. EDTA, may be used to promote double and/or multi strand denaturation under acidic conditions. The temperature required for denaturation at low pH is ambient to mildly elevated (e.g. 4°C to 50°C, preferably ambient temperature).

According to the present invention, RNA containing double- and/or multi-stranded nucleic acid impurities can be incubated at low pH to disassociate double- and/or multi-stranded species, then the pH is increased to more than pH 5 to pH 7. This increase of pH confers long-term stability to RNA, while impurities do not reform, or do not reform fully, so that potential immune responses triggered by such impurities are not activated upon administration of the RNA *in vivo.*

Further, RNA containing double- and/or multi-stranded nucleic acid impurities can be incubated at low pH to disassociate double- and/or multi-stranded species, then the pH is increased to pH 5 to pH 7, pH 5 in case the low pH is less than pH 5, and the preparation is purified to remove fragments produced by double and/or multi strand disassociation. Alternatively, RNA containing respective impurities can be incubated at low pH, then purified to remove fragments produced by double and/or multi strand disassociation, wherein the purification is carried out in the pH range used for denaturation (e.g. pH 2 to pH 4).

The present invention can exploit the unusual pH range used for disruption of dsRNA and RNA-DNA double- and/or multi-stranded structures and for binding of RNA to be prepared to chromatographic media (porous particles, nanofibers, membranes, monoliths) used in ion exchange (cation and anion exchange), size exclusion, reversed phase, hydrophobic interaction, or multi-modal chromatographic supports. Furthermore, molecular weight cut-off devices, e.g. tangential-flow filtration devices can be used.

In this context, anion exchange ligands can be used in an unusual pH range. Due to the strongly negative charge carried by the phosphate backbone, the RNA molecule, such as mRNA, is strongly negatively charged at pH 1.6 and above. Anion exchange ligands e.g. quaternary amine (QA), tertiary amine (e.g. DEAE), secondary amine (e.g. ethylenediamine), primary amine, or multi-modal ligands comprising at least one anion exchange modality, are positively charged in a pH range of pH 1 to pH 11 or pH 1 to pH 13 (weak anion exchangers and strong anion exchangers, respectively). RNA can thus be bound to a positively charged ligand at a pH range as low as pH 1 to pH 2. This is not common practice, and is indeed counterintuitive, because the use of anion exchange ligands in the art is at neutral-to-basic conditions, with the lowest reported pH value for use of anion exchanger for purification of mRNA being pH 5. The present invention exploits the complementarity of charges of RNA and anion exchange ligands in an unusual pH range of pH 1 to pH 5, specifically pH 2 to pH 4. RNA fragments annealed to target RNA which form dsRNA structures, and residual DNA fragments forming RNA-DNA structures, will be separated from the main ssRNA chain using a salt gradient or pH gradient in a selected buffer (e.g. citrate, acetate, glycine, glycylglycine, malate, maleate, phosphate, succinate, or formate buffers). As an additional aspect, any undissociated double- and/or multi-stranded structures may bind to anion exchange ligands differentially from ssRNA structures, thus providing an additional layer of purification control. Additives that facilitate denaturation as known in the art, e.g. chaotropes, e.g. urea or guanidine, or chelating agents, e.g. EDTA or citrate, or organic solvents, e.g. acetonitrile or ethanol, may be used to promote double and/or multi strand denaturation in mobile phases, thereby further increasing the selectivity of the method. The temperature of separation may be optimized to lead to an optimal balance of chromatographic separation and double and/or multi strand denaturation.

Alternatively, cation exchange ligands can be used for purification of target RNA after low pH-treatment. This is counterintuitive, because e.g. mRNA is strongly negatively charged in a pH range of pH 1.9 to pH 13, whereas cation exchange chromatographic supports bind positively charged species.

Further, size exclusion chromatography can be used to separate single stranded fragments produced upon pH-denaturation of double- and/or multi-stranded impurities from the desired RNA target molecule. Size exclusion chromatography can be performed at the pH range used for denaturation (e.g. pH 2 to pH 4) or at neutral/mildly acidic pH (e.g. pH 5 to pH 7, pH 5 in case the pH range used for denaturation is less than pH 5). Small fragments which had formed double- and/or multi-stranded species by hybridization to parent RNA, as well as 3' extended dsRNA species are separated from target RNA by size.

Furthermore, reversed phase chromatography or hydrophobic interaction chromatography can be used to separate single stranded fragments produced upon pH-denaturation of double- and/or multi-stranded impurities from the desired RNA target molecule, based on differential hydrophobicity of fragments compared to target RNA. Reversed phase chromatography and hydrophobic interaction chromatography can be performed at the pH range used for denaturation (e.g. pH 2 to pH 4) or at neutral/mildly acidic pH (e.g. pH 5 to pH 7, pH 5 in case the pH range used for denaturation is less than pH 5).

In all embodiments using chromatographic purification, the latter is not limited to specific chromatographic media, wherein any format containing a suitably charged polymer that facilitates binding at target pH range can be used, e.g. monoliths, membranes, filters, nanofibers, or porous particles.

Further, tangential flow filtration, operated in the pH range used for denaturation (e.g. pH 2 to pH 4) or in an increased pH range (e.g. pH 5 to pH 7, pH 5 in case the pH range used for denaturation is less than pH 5) can be used to separate fragments/impurities produced by pH-denaturation of double- and/or multi-stranded nucleic acids from target RNA molecules.

The figures show:
Figure 1:
   (A) Chemical structure of dsRNA and (B) protonation equilibria of nucleosides. Boxes denote protonation state achieved by low pH treatment.
Figure 2:
   pH treatment of mRNA reduces dsRNA content without affecting mRNA stability. Re-neutralization to pH 6 does not lead to reformation of dsRNA. Left: dot-blot (J2 antibody). Right: Agarose gel electrophoresis.
Figure 3:
   A. Denaturation of dsRNA with low pH is temperature-dependent. eGFP (995 nt) was incubated at different pH values in the range of 2.9 - 4.1 for 30 min at 4 °C, room temperature (RT) and 40°C. Dot-blot with J2 antibody was performed to determine the amount of dsRNA. B. Denaturation of long dsRNA chains requires elevated temperature in conjunction with low pH for denaturation. Magi2 dsRNA (1000 bp full complement dsRNA) was incubated at 50°C for 20 min at pH 3 (sample 1) or pH 6 (sample 3), then buffer-exchanged to pH 3 (samples 2 and 4, respectively). Denaturation was monitored by A) PAGE and B) J2 dot-blot.
Figure 4:
   Exposure of affinity-purified mRNA to low pH for up to 60 min does not cause degradation of mRNA. A. eGFP (995 nt) was incubated at pH 3 for i) 15, ii) 30 or iii) 60 min, then pH was increased to pH 6. Fragment analyzer (BioAnalyzer, Agilent) was used to determine the degree of fragmentation directly after neutralization to pH 6 (i-iii) or after 1 day storage of same samples at 4-8 °C (iv-vi). B. Agarose gel electrophoresis of samples i-vi. Lane 1: Riboruler HR, Lane 2: pH 3, 15 min, Lane 3: pH 3, 30 min, Lane 4: pH 3, 60 min, Lane 5-7: samples from lanes 2-4 analyzed after 1 day storage at 4-8 °C.
Figure 5:
   Exposure of IVT mixture containing mRNA to low pH leads to a decrease in dsRNA content as measured by J2 dot-blot.
   i) IVT mixture containing mRNA was incubated at pH 3 for 30 min at T (25 °C)
   ii) IVT mixture containing mRNA was analyzed in IVT reaction buffer (pH 7.9)
Figure 6:
   An example of low pH treatment of mRNA coupled with removal of dsRNA structures by chromatography. eGFP (995 nt) was incubated in 100 mM glycine, pH 3.0 at ambient temperature for 25 min. Thereafter, pH was increased to pH 6 with 100 mM glycine pH 10. Sample was then loaded onto CIM PrimaH column (prototype multi-modal column combining the elements of a weak anion-exchanger, aromaticity, and hydrogen bonding) at pH 5 and eluted with pH gradient (pH 5 - pH 7.5) made with 20 mM citrate, 20 mM Na-phosphate, 10 mM EDTA buffer system. A: elution chromatogram of pH 3-incubated eGFP mRNA on CIM PrimaH. Red: 260 nm, blue: 280 nm, green: linear gradient, grey: pH trace, brown: conductivity. B: PAGE gel of starting material, CIM PrimaH elution fractions 1 and 2 and cleaning-in-place (high pH wash, CIP). C: J2 dot-blot of eGFP (L), and CIM PrimaH elution fractions 1 and 2 and cleaning-in-place (high pH wash, CIP). D: AGE of gel starting material, CIM PrimaH elution fractions 1 and 2 and cleaning-in-place (high pH wash, CIP).
Figure 7:
   An example of low pH treated mRNA for removal of dsRNA structures with cation exchange chromatography. eGFP (995 nt) was either incubated in 100 mM glycine, pH 3.0 at ambient temperature for 20 min or prepared directly for cation exchange chromatography. Samples were diluted in mobile-phase A 50 mM glycine pH 3 and loaded onto CIM SO3 column at pH 3 and eluted with pH gradient pH 3 - pH 10. A: elution chromatogram of eGFP mRNA on CIM SO3. Green: untreated mRNA, red: pH 3 treated mRNA, B: Polyacrylamide gel electrophoresis (PAGE) of starting material (either pre-treated in pH 3, or without pre-treatment), flow-through (FT) fraction and elution fractions E1, E2 and CIP. C: J2 dot-blot of corresponding fractions.
Figure 8:
   Low pH treatment in conjunction with chromatographic separation leads to removal of residual DNA template. eGFP (995 nt) was incubated in 100 mM glycine, pH 3.0 at ambient temperature for 25 min or not treated with low pH. Both samples were then purified on CIM PrimaH using a pH gradient. Main elution fraction was analyzed for the presence of residual DNA plasmid template by RNAse treatment and AGE. Lane 1: GeneRuler, Lane 2: 50 ng eGFP DNA template, Lane 3: 30 ng eGFP DNA template, Lane 4: 10 ng eGFP DNA template, Lane 5: 5 ng eGFP DNA template, Lane 6: 2 ng eGFP DNA template, Lane 7: 1 ng eGFP DNA template, Lane 8: mRNA purified with Oligo dT (starting material), Lane 9: PrimaH elution fraction of pH 3-treated mRNA, Lane 10: PrimaH elution fraction of untreated mRNA

The present invention will be further illustrated by the following example without being limited thereto.

### Example

mRNA encoding for eGFP (enhanced green fluorescent protein; 995 nucleotides) was produced in IVT (*in vitro* transcription) reaction and purified by Oligo-dT affinity chromatography, which does not remove dsRNA (double-stranded RNA). Dot-blot analysis with J2 antibody, specific for dsRNA sequences of at least 40 base pairs, confirmed presence of dsRNA impurity in the sample.

The pH range required for denaturation of dsRNA was tested by incubation of mRNA in a wide pH range (2.9 - 4.1; glycine buffer was used at specified pH values) at room temperature. Samples were incubated at room temperature for 30 min before loading onto AGE (agarose gel electrophoresis; 100 ng) or dot-blot assay (800 ng). Incubation of mRNA at pH 2.9-4.1 did not reduce the intensity of the main mRNA AGE band whereas the intensity of dot-blot signal decreased with decreasing pH. The signal was strongest at pH 6, and strong signal was still observed for pH 3.9-4.1. Signal decreased significantly at pH 3.5 and was not visible for pH 3.2 and below, suggesting that dsRNA structures were disassociated, while mRNA was intact.

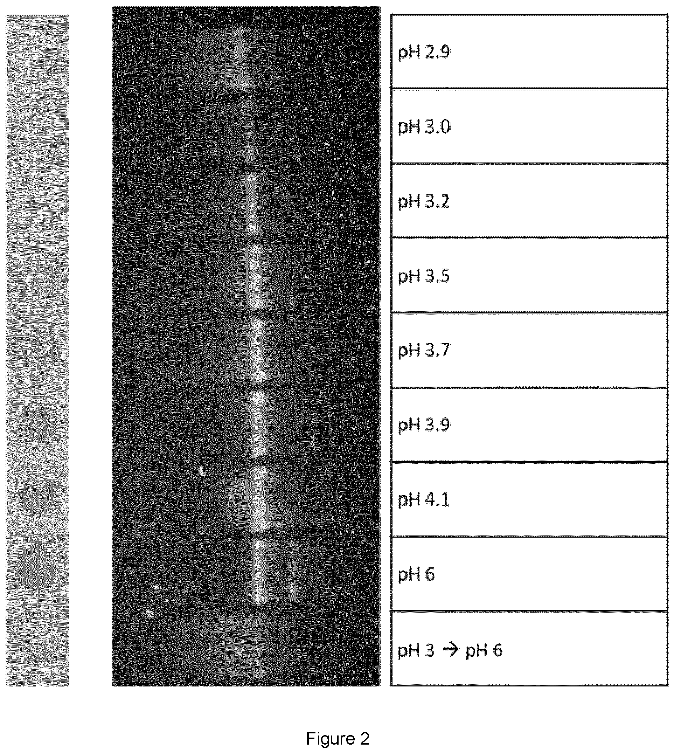

mRNA was then incubated at pH 3 for 20 min, then increased to pH 6; AGE results showed appearance of lower molecular weight bands upon increase to pH 6, whereas dot-blot showed no signal, suggesting that dsRNA structures were not reformed (Figure 2).

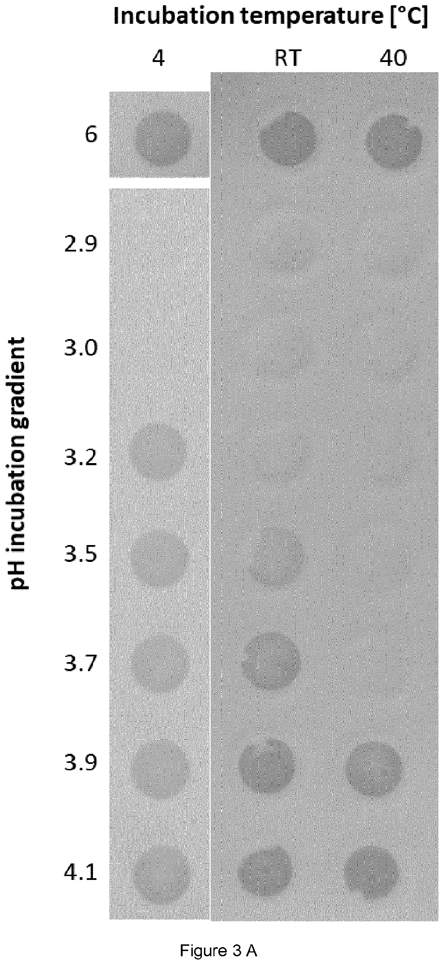

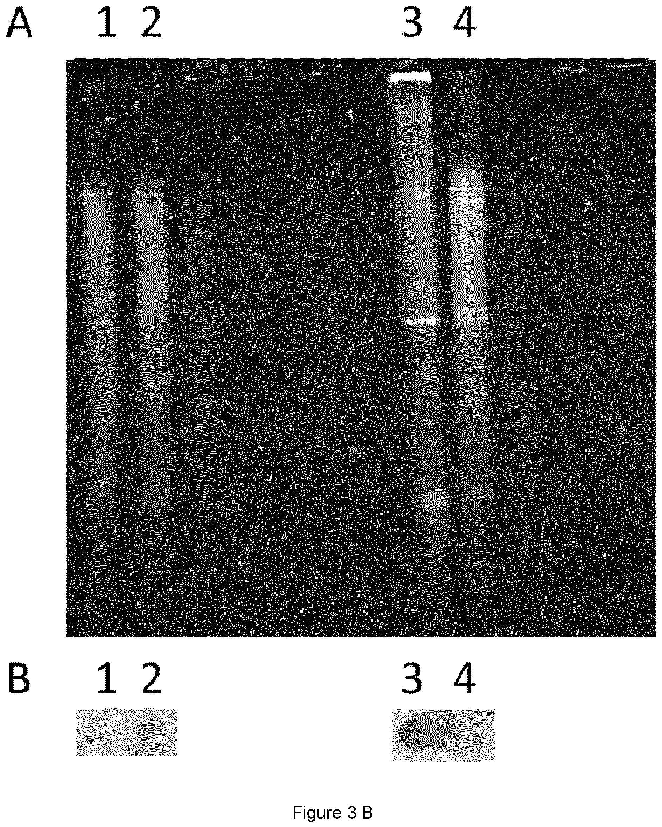

Temperature dependence of dsRNA denaturation was demonstrated by incubating eGFP (995 nt) at different pH values in range of 2.9 - 4.1 for 30 min at 4°C, room temperature (RT) and 40°C. Dot-blot with J2 antibody was performed to determine the amount of dsRNA and demonstrated a temperature- and pH dependent decrease in dsRNA signal. dsRNA signal was still detected at pH 3.2 at 4°C, whereas at 40°C, the dsRNA signal was only detected at pH 3.9 or higher (Figure 3).

To demonstrate that double strand denaturation requires pH rather than temperature, a fully complementary dsRNA sequence (1000 base pairs) Magi2 was incubated at either pH 3 or pH 6 at 50°C for 20 min. At pH 6, high temperature (50°C) was not sufficient to denature the double strand as shown by J2 dot-blot (Figure 3B), whereas at pH 3 dsRNA signal disappeared. Upon buffer exchange into pH 3, dsRNA signal disappeared (Figure 3B, sample 4).

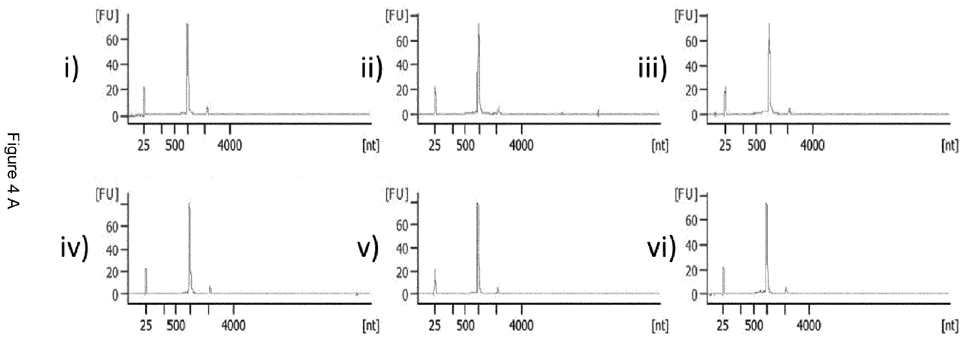

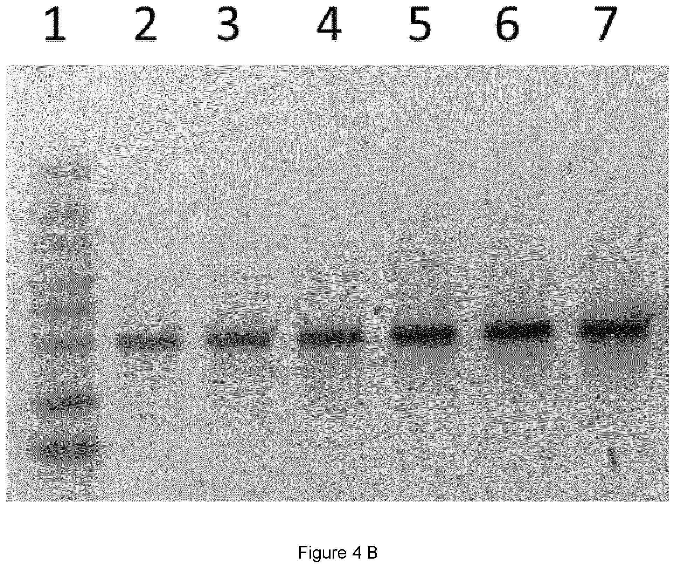

To demonstrate that low pH treatment does not affect stability of mRNA, eGFP (995 nt) was incubated pH 3 for 15, 30 and 60 min at room temperature before neutralization to pH 6. Fragmentation was then measured by BioAnalyzer and AGE (Figure 4A and 4B, respectively). No significant degradation was observed by either method, indicating that exposure of mRNA to low pH for the duration as required for denaturation of dsRNA, does not lead to degradation of mRNA.

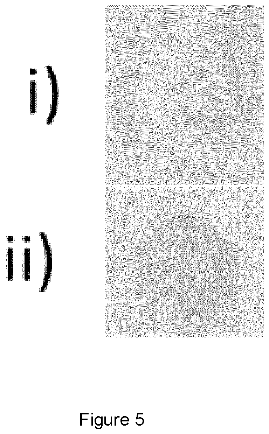

To demonstrate the generality of the approach, unpurified IVT reaction mixture containing eGFP mRNA was then either incubated at pH 3 or not treated, and dsRNA signal measured by J2 dot-blot, in absence of RNA purification. Dot-blot demonstrated the disappearance of dsRNA signal at pH 3 (Figure 5i), but not in absence of pH treatment (Figure 5ii).

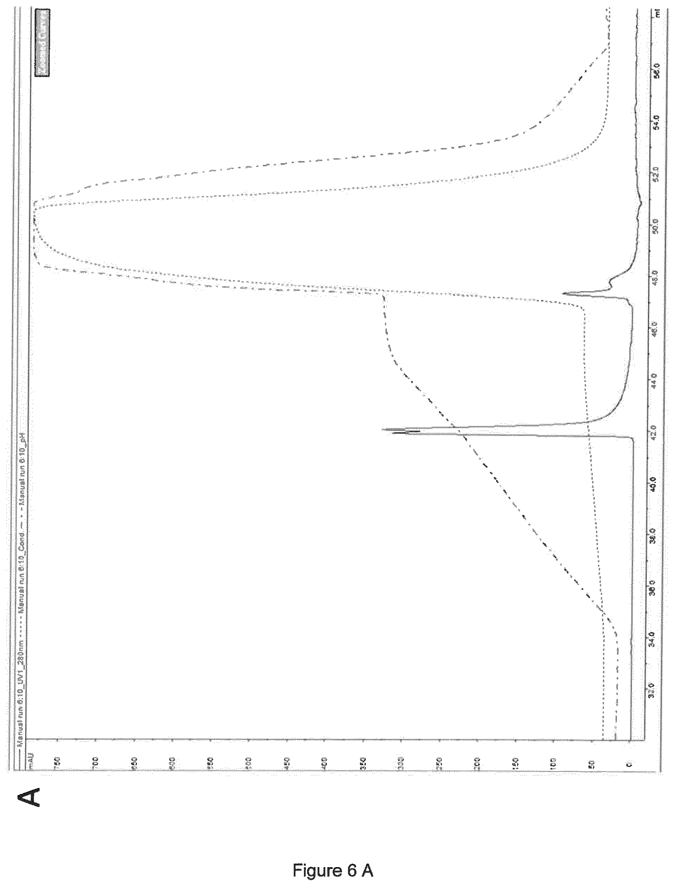

To demonstrate the purification of low pH-treated mRNA, mRNA encoding for eGFP was incubated in 100 mM glycine, pH 3.0 at ambient temperature for 20 min. The pH of the sample was then increased to pH 6 with a glycine buffer (pH 10) and the sample was then loaded onto a prototype multi-modal column combining the elements of a weak anion-exchanger, aromaticity, and hydrogen bonding (PrimaH) at pH 5 and eluted with pH gradient (pH 5 - pH 7.5) made with 20 mM citrate, 20 mM Na-phosphate, 10 mM EDTA buffer system. (Figure 6 A). Two elution fractions (1 and 2) were analyzed by dot-blot with J2 antibody.

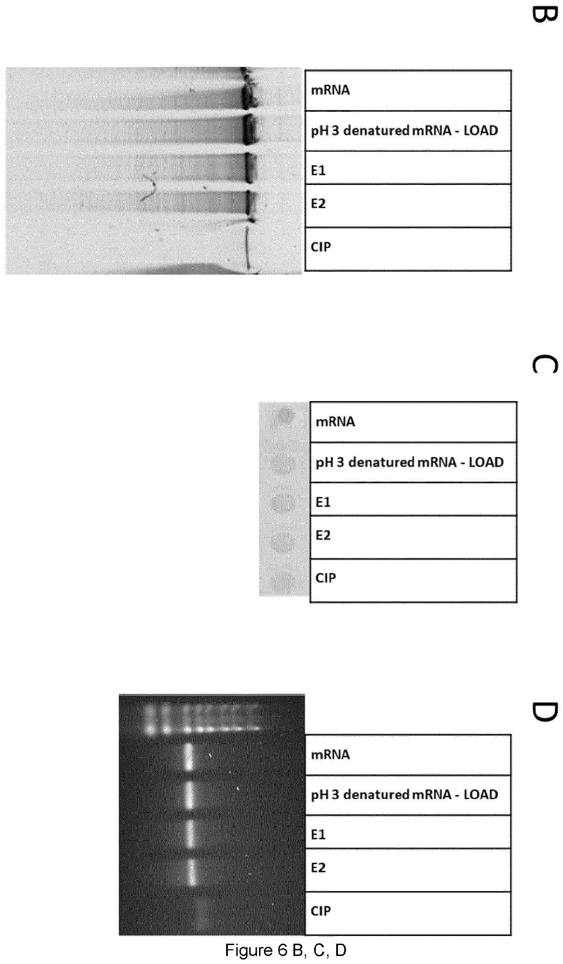

Results demonstrated strong binding of mRNA to PrimaH when incubated at pH 3 (Figure 6). pH treatment of mRNA led to a significant reduction in dsRNA content in elution fractions, as demonstrated by J2 dot-blot, fractions 1 and 2 (Figure 6).

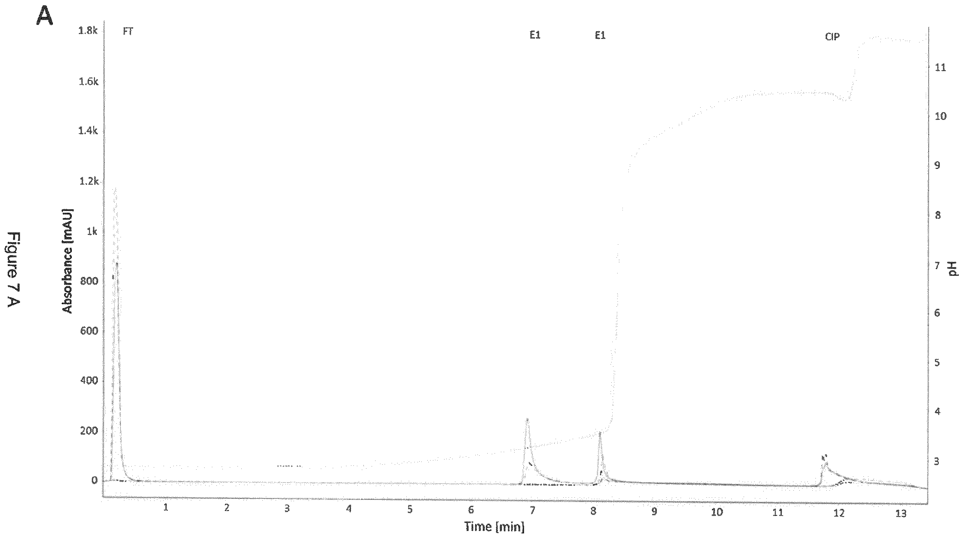

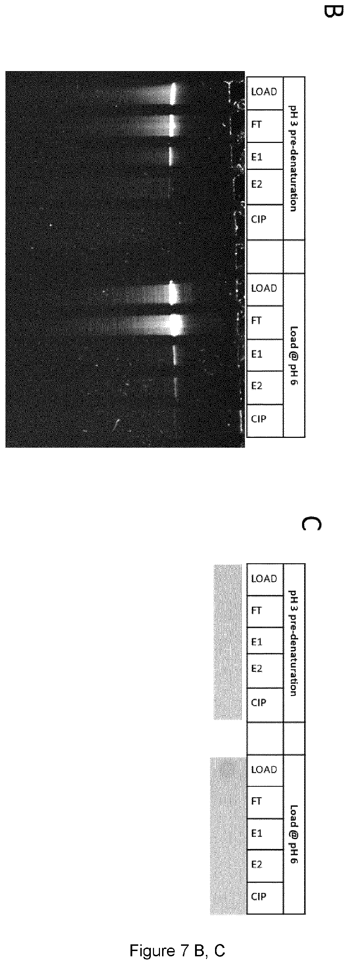

To demonstrate the purification of low pH-treated mRNA with cation exchanger, mRNA encoding for eGFP was either incubated in 100 mM glycine, pH 3.0 at ambient temperature for 20 min, or untreated. Both samples were separately diluted in mobile phase A (MPA; 50 mM glycine pH 3) and loaded onto a CIM (Convective Interaction Media) SO3 column and eluted with a pH gradient 3 - 10 (Figure 7 A). The starting material (mRNA treated or untreated with low pH), flow-through fraction and two elution fractions (1 and 2) were analyzed by PAGE and dot-blot with J2 antibody (Figure 7 B, C). Results demonstrated that both pre-treatment with pH 3, or dilution of sample in MPA (50 mM glycine pH 3), leads to denaturation of dsRNA as demonstrated by a decrease in dot-blot signal. ssRNA elutes in flow-through; dot-blot indicates absence of dsRNA (Figure 7C).

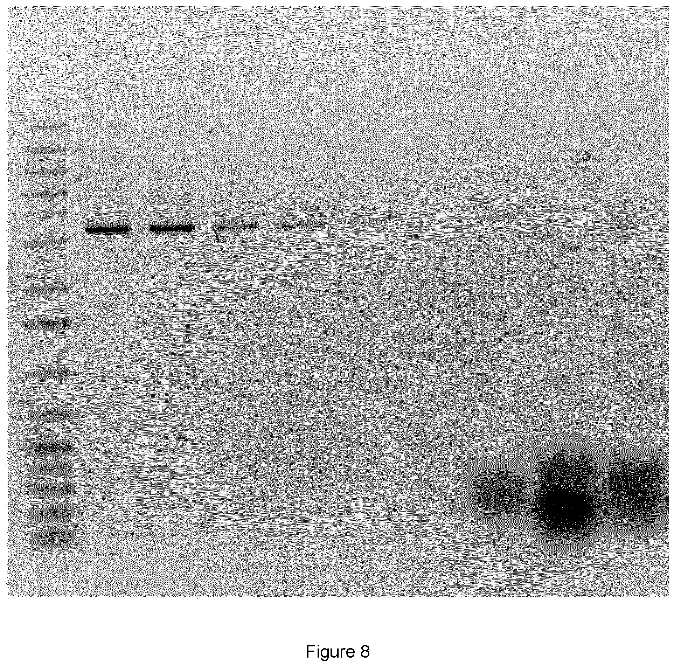

To demonstrate that low pH treatment in conjunction with chromatographic separation leads to removal of residual DNA template, eGFP (995 nt) was incubated in 100 mM glycine, pH 3.0 at ambient temperature for 25 min before neutralization; control was not treated with low pH. Both samples were then purified on CIM PrimaH using a pH gradient as described above. Main elution fraction was analyzed for the presence of residual DNA plasmid template by treatment of 10 µg of mRNA with RNAse A (to enzymatically digest mRNA); product of RNAse A digestion of 10 ug mRNA was loaded onto AGE. RNAse A selectively digests RNA, but not DNA, and residual DNA signal is compared to a standard dilution of parent linearized plasmid (range 50 ng - 1 ng). When PrimaH elution of pH 3-treated mRNA was analyzed by AGE using this methodology, no signal for residual plasmid was detected. PrimaH elution of pH-untreated mRNA showed the presence of 2-5 ng DNA plasmid / 10 µg mRNA (Figure 8).

## Claims

1. A method for the removal of double- and/or multi-stranded nucleic acid impurities from an RNA preparation, comprising the steps of:
(a) incubating the RNA preparation at a pH in the range of pH 1 to pH 5, and
(b) subjecting the RNA preparation to purification to remove fragments produced by the dissociation of the double- and/or multi-stranded nucleic acid impurities.

2. The method according to claim 1, wherein in step (a), the RNA preparation is incubated for 25 seconds to 1 hour.

3. The method according to claim 1 or claim 2, wherein step (a) is performed in a buffer comprising a denaturing agent and/or a chelating agent.

4. The method according to any one of claims 1 to 3, wherein step (a) is performed at a temperature of 4°C to 50°C.

5. The method according to any one of claims 1 to 4, wherein step (a) is performed as part of an affinity chromatography step, in which the RNA preparation is loaded onto the affinity chromatography medium at a pH in the range of pH 6 to pH 8, and subsequently the pH is lowered to a pH in the range of pH 1 to pH 5.

6. The method according to any one of claims 1 to 5, wherein
(i) the RNA preparation is subjected to the purification at the pH used in step (a), or
(ii) the pH of the RNA preparation is increased to a pH in the range of a pH higher than the pH used in step (a) to pH 5, provided step (a) is not already performed at pH 5, prior to the purification, or
(iii) the pH of the RNA preparation is increased to a pH in the range of more than pH 5 to pH 7, prior to the purification.

7. The method according to any one of claims 1 to 6, wherein the purification is effected by a technique, selected from the group consisting of cation exchange chromatography, anion exchange chromatography, size exclusion chromatography, reversed phase chromatography, hydrophobic interaction chromatography, multi-modal chromatography, affinity chromatography, IMAC (immobilized metal affinity chromatography), molecular weight cut-off filtration, and precipitation/extraction techniques.

8. The method according to any one of claims 1 to 6, wherein
(i) the purification is effected by a technique, selected from the group consisting of cation exchange chromatography, anion exchange chromatography, size exclusion chromatography, reversed phase chromatography, hydrophobic interaction chromatography, multi-modal chromatography, affinity chromatography, and IMAC (immobilized metal affinity chromatography), and
(ii) steps (a) and (b) are performed concurrently while the RNA preparation is bound to the chromatographic medium.

9. The method according to claim 7 or claim 8, wherein the technique is anion exchange chromatography that is performed at a pH in the range of pH 1 to pH 5 using an anion exchange ligand, selected from the group consisting of quaternary amines (QA), tertiary amines, secondary amines, and primary amines.

10. The method according to claim 9, wherein the anion exchange ligand is diethylaminoethyl (DEAE) or dimethylaminoethyl (DMEA).

11. The method according to claim 7 or claim 8, wherein the technique is cation exchange chromatography that is performed at a pH in the range of pH 1 to pH 5 using a cation exchange ligand, selected from the group consisting of cation exchange ligands containing sulfonate groups, cation exchange ligands containing sulfate groups, and cation exchange ligands containing carboxyl groups.

12. The method according to claim 7 or claim 8, wherein the technique is multi-modal chromatography that is performed at a pH in the range of pH 1 to pH 5 using a multi-modal ligand comprising multiple types of chemical residues, selected from the group consisting of affinity residues, hydrophobic interaction residues, ion exchange residues, hydrogen bonding residues, metal chelating residues, aromatic residues, and combinations thereof.

13. The method according to claim 7 or claim 8, wherein the technique is affinity chromatography that is performed at a pH in the range of pH 1 to pH 5 using an affinity ligand.

14. The method according to any one of claims 1 to 13, wherein the RNA to be prepared in the RNA preparation is selected from the group consisting of messenger RNA (mRNA), self-amplifying RNA (saRNA) and circular RNA (circRNA).

15. The method according to any one of claims 1 to 14, wherein the double- and/or multi-stranded nucleic acid impurities are non-single-stranded nucleic acid structures, selected from the group consisting of double-stranded RNA (dsRNA), double-stranded DNA (dsDNA), RNA-DNA heteroduplex structures, and multi-stranded homo- or heteromeric nucleic acid structures.
